# EUROPÄISCHE PATENTSCHRIFT

(11) **EP 2 103 270 B1**
(45) Veröffentlichungstag und Bekanntmachung des Hinweises auf die Patenterteilung: **27.06.2012**
(21) Anmeldenummer: 08152941.4
(22) Anmeldetag: 18.03.2008
(51) Int. Cl.: A61B 19/00

(54) **System für navigations-unterstützte Schulteroperationen zur Positionierung navigierter Behandlungsgeräte bezüglich eines Knochens**
System for navigation-supported shoulder operations for positioning navigated treatment devices relative to a bone
Système pour opérations de l'épaule assistées par navigation destiné au positionnement d'appareils de traitement navigué relatif à un os

(43) Veröffentlichungstag der Anmeldung: 23.09.2009
(73) Patentinhaber: BrainLAB AG, 85622 Feldkirchen (DE)
(72) Erfinder: Neubauer, Timo, 85586, Poing / Angelbrechting (DE); Fessler, Julia, 85622, Feldkirchen (DE)
(74) Vertreter: Schwabe - Sandmair - Marx

(56) Entgegenhaltungen:
- EP-A- 1 647 236
- EP-A- 1 854 425
- DE-A1- 10 145 587
- US-A1- 2003 153 829
- US-A1- 2005 245 808
- US-A1- 2006 241 388

## Beschreibung

### Gebiet der Erfindung

Die vorliegende Erfindung bezieht sich auf ein System für navigations-unterstützte Operationen, insbesondere auf ein System für navigations-unterstützte Schulteroperationen, in denen die humerale Komponente teilweise durch eine Prothese ersetzt wird. Des Weiteren bezieht sich die vorliegende Offenbarung auf ein Navigationsverfahren zur Positionierung navigierter Behandlungsgeräte bezüglich eines Knochens.

### Stand der Technik

In den letzten Jahren wurden mit großem Erfolg navigations-unterstützte Operationssysteme entwickelt, um den Operateur bei seiner Arbeit effektiv zu unterstützen. Diese navigations-unterstützten Operationssysteme erlauben es, die während einer Operation eingesetzten Behandlungsgeräte exakt zu positionieren (navigierte Behandlungsgeräte). Dadurch hat sich das Risiko, das für einen Patienten mit einer Operation verbunden ist, deutlich senken lassen. Auch die Qualität der Behandlungsergebnisse stieg deutlich an, und die Lebensqualität der Patienten nach einer Operation konnte erheblich verbessert werden.

Bei den bekannten Navigationssystemen werden an Behandlungsgeräten, beispielsweise an chirurgischen Instrumenten, Marker wie aktive Signalgeber oder Reflektoren angebracht. Auch am Patienten werden Marker (z.B. aktive Signalgeber oder Reflektoren) angebracht.

Wird nun mit einer Kameraanlage, die dazu in der Lage ist, räumliche Positionswerte zu erfassen, das Operationsgebiet überwacht und die Marker (aktive Signalgeber oder Reflektoren) an Patient und Behandlungsgerät gleichzeitig erfasst, so können ihre Positionen zueinander und dadurch wiederum die Position des Behandlungsgerätes am Patienten optimal gesteuert werden.

Navigations-unterstützte Operationssysteme werden insbesondere auch bei Operationen verwendet, in deren Verlauf der Patient eine Prothese eingesetzt erhält. So werden heute Kniegelenksoperationen und Hüftgelenksoperationen mit Protheseneinsatz standardmäßig durchgeführt.

Bei Schulteroperationen hingegen ist derselbe Grad an routinemäßigem Einsatz noch nicht erreicht worden. Dies liegt unter anderem daran, dass sich im Schulterbereich besonders viele Nerven und Gefäße befinden, die während -einer navigierten Operation durch die hier notwendige Anbringung von Referenzeinheiten zur Erfassung der Schulter- oder Oberarmlage relativ zum Behandlungsgerät verletzt werden könnten, was zu einer Einschränkung -der Mobilität des Patienten im Schulter- und Armbereich nach der Operation- führen könnte. Auch handelt es sich bei dem Oberarmknochen des Schultergelenkes um einen im Vergleich zu Ober- und Unterschenkel wesentlich kleineren Knochen, der weniger starken Belastungen standhalten kann.

Wird bei einer Schulteroperation die humerale Komponente durch eine Prothese ersetzt, so wird standardmäßig meist ein nicht-navigations-unterstütztes Operationsverfahren angewendet. Nachdem sich operativ eine Zugriffsmöglichkeit auf den Oberarmknochenkopf (engl. "humeral head") verschafft wurde und dieser luxiert wurde, wird eine Pilotöffnung (engl. "pilot hole") in den oberen Bereich des Humurus gebohrt, und der humerale Kanal wird ausgefräst. Anschließend wird eine Schnittführung an -einer Stange,- die in dem ausgehöhlten Kanal angebracht ist, befestigt und -orientiert, um den Oberarmknochenkopf definiert abzuschneiden. Schließlich wird der humerale Kanal für die Implantierung einer Prothese vorbereitet (z.B. durch Ausreiben etc.).

Aus der WO 2006/050010 A2 ist ein navigiertes System und Verfahren für Schulteroperationen bekannt. Gemäß diesem Verfahren werden positionsgebende Reflektoren und Marker, die Bestandteil einer Tracking-Einheit (engl. "tracking unit") sind, am Schulterblatt fest verschraubt. Nach der Operation wird diese Verschraubung wieder entfernt. Es gilt jedoch zu bedenken, dass jeder Verschraubungsvorgang letztlich einen Knochen verletzt. Es besteht die Gefahr, dass ein Knochen auch instabil wird und bricht. Des Weiteren besteht die Gefahr von Infektionen durch zusätzliche Inzisionen zur Anbringung von Referenzeinheiten. Deshalb sind Bohrungen in Knochen nach Möglichkeit zu vermeiden.

EP 647236 A1 zeigt eine Vorrichtung und ein Verfahren zur Lageüberprüfung von Markern. In dem Verfahren wird eine Positionsänderung mindestens eines an einem ersten Objekt angebrachten ersten Markers oder Referenzsterns relativ zu dem ersten Objekt erkannt, wobei die Lage mindestens eines ersten charakteristischen Punktes des ersten Objekts mittels des daran befestigten ersten Markers oder Referenzsterns ermittelt wird, die Lage mindestens eines zweiten charakteristischen Punktes eines mit dem ersten Objekt verbundenen zweiten Objekts ermittelt wird und aus der ermittelten Lage der mindestens zwei charakteristischen Punkte bestimmt wird, ob sich die Position des mindestens einen ersten Markers oder Referenzsterns relativ zu dem ersten Objekt geändert hat. Eine Schneidführung ist weder in dem Verfahren noch in der entsprechenden Vorrichtung vorgesehen.

DE 10145587 A1 behandelt ein Verfahren und eine Vorrichtung zur Prüfung eines Markierungselements auf Verrückung gegenüber einer Haltestruktur, an der das Markierungselement fixiert ist. Dabei wird das Markierungselement zur Positionsbestimmung bei der navigativen Chirurgie verwendet und ein Orientierungspunkt, welcher in einer eindeutigen Beziehung zu der Haltestruktur steht, gewählt sowie die Position des Orientierungspunkts in einem Referenzsystem des Markierungselements überwacht. Auch dieser Schrift kann eine Schneidführung nicht entnommen werden.

EP 854425 A1 zeigt ein Verfahren zur medizintechnischen Positionsbestimmung mit redundantem Positionserfassungseinrichtungen sowie einer Prioritätsgewichtung für die Positionserfassungseinrichtung. Ferner betrifft diese Schrift auch ein Verfahren zum Steuern eines medizintechnischen Manipulators, insbesondere eines Roboters, der mit Hilfe von Positionsinformationen gesteuert wird die mit einem solchen Positionsbestimmungsverfahren erhalten wurden. Dieser Roboter umfasst jedoch laut der entsprechenden Offenbarung nicht eine Schneidführung.

US2003/0153829A1 zeigt ein computerassistiertes Navigationssystem für Hüftersatzoperationen. Das System umfasst ein Lokalisierungssystem mit einem daran angeschlossenen Computer zur Positionsverfolgung von Objekten in einem generischen Computermodell der Geometrie einer Hüfte eines Patienten. Dabei wird ein Acetabulum-Implantat in einer gewünschten Lagebeziehung zu einer Ebene der Hüfte eingestellt, ohne sich dabei auf zuvor erhaltene radiologische Daten zu beziehen, indem die Lagebeziehung des Acetabulum-Implantats an der genannten Hüftebene ausgerichtet wird. Auch diese Veröffentlichung zielt nicht auf die Anwendung einer Schneidführung.

Des Weiteren ist bekannt, bei Schulteroperationen am Oberarmknochen eine Befestigungseinheit anzubringen, an der an verschiedenen Positionen eine Navigationseinheit bzw. Tracking-Einheit anbringbar ist. Auf diese Weise kann eine einzige Tracking-Einheit so angebracht werden, dass sie für ein für Navigationszwecke genutztes Kamerasystem besonders gut sichtbar ist.

Es ist die Aufgabe der vorliegenden Erfindung, ein verbessertes System bereitzustellen, das für navigations-unterstützte Schulteroperationen, in denen die humerale Komponente teilweise durch eine Prothese ersetzt wird, geeignet ist, und bei dem gleichzeitig die oben beschriebenen Risiken für den Patienten gesenkt werden. Insbesondere ermöglicht das erfindungsgemäße System weitere Behandlungsschritte, die mit Exaktheit durchgeführt werden müssen, navigations-unterstützt und damit positionsgenauer durchzuführen.

Die Aufgabe wird gelöst durch das System gemäß Anspruch 1. Die Unteransprüche definieren bevorzugte Ausführungsformen der Erfindung.

### Beschreibung der Erfindung

Ein System für navigations-unterstützte Schulteroperationen, in denen die humerale Komponente teilweise durch eine Prothese ersetzt wird, weist eine erste Tracking-Einheit (engl. "tracking unit") auf, die angepasst ist, um an einem Abschnitt eines Knochens befestigt zu werden, der während der Operation entfernt werden soll. Dadurch, dass die erste Tracking-Einheit an einem Abschnitt des Knochens befestigt wird, der während der Operation entfernt werden soll, wird vermieden, dass durch die Befestigung der ersten Tracking-Einheit die Gefahr einer Instabilität des Knochens oder die Gefahr einer Infektion durch weitere Inzisionen zusätzlich zu den ohnehin gegebenen Risiken der Operation besteht.

Weiterhin weist das System eine Detektionseinheit auf, die angepasst ist, um Landmarken auf dem Knochen zu detektieren. Dabei kann eine gewöhnliche Detektionseinheit z.B. in Form eines Pointers verwendet werden. Es ist möglich, besonders charakteristische Stellen auf dem Knochen als Landmarken zu detektieren. Es ist weiterhin möglich, nicht nur einzelne Punkte zu detektieren, sondern einen Scan mit vielen Punkten durchzuführen, um genaue Kenntnisse von der äußeren Form des Knochens zu erhalten. In beiden Fällen wird im Rahmen dieser Anmeldung von "Landmarken" gesprochen.

Das System weist weiterhin eine zweite Tracking-Einheit auf, die angepasst ist, um an einem anderen Abschnitt desselben Knochens angebracht zu werden. Bei diesem anderen Abschnitt desselben Knochens handelt es sich bevorzugt um einen Abschnitt, der nicht während der Operation entfernt werden soll. Es kann sich zum Beispiel um einen Abschnitt handeln, an dem später das Implantat befestigt wird, und die zweite Tracking-Einheit wird in diesem Beispiel intramedullär, d.h. mit einer Einheit,- die in den Markkanal des Knochens hineinreicht, an diesem befestigt. Grundsätzlich ist es möglich, als zweite Tracking-Einheit die bekannten Tracking-Einheiten einzusetzen, d.h. Einheiten, die mit aktiven Signalgebern oder Reflektoren versehen sind.

Das System umfasst weiterhin eine Kameraeinheit, die die erste und zweite Tracking-Einheit gleichzeitig erkennt, um deren jeweilige Lage zu bestimmen. Dazu werden erfindungsgemäß mittels der Kameraeinheit (z.B. Kamera) Marker der Tracking-Einheiten detektiert. Die Tracking-Einheiten umfassen- mindestens zwei Marker, die in fester und vorbestimmter relativer Lage zueinander angeordnet sind und insbesondere mechanisch verbunden sind. Die Marker können passive oder aktive Marker sein, wobei die passiven Marker Markersignale (z.B. Wellen und/oder Strahlung) reflektieren, die in ihre Richtung ausgesendet werden, und die aktiven Marker selbst Ursprung der Signale (z.B. Strahlung und/oder Wellen) sind. Die von den (aktiven oder passiven) Markern ausgehenden Signale, bei denen es sich z.B. um Wellensignale oder Strahlungssignale handeln kann, werden von der Kameraeinheit detektiert. Um eine Position der Tracking-Einheit relativ zu der Kameraeinheit festzulegen, wird dabei vorzugsweise die Tracking-Einheit bewegt, um der Kameraeinheit verschiedene Ansichten der Tracking-Einheit zu bieten. Auf dieser Grundlage kann dann in bekannter Weise die relative Lage der Tracking-Einheit relativ zu der Kameraeinheit, insbesondere in einem im Raum ruhenden Bezugssystem bestimmt werden. In diesem Zusammenhang wird auf die DE 196 29 615 A1 und die entsprechende US-Veröffentlichung 6,351,659 hingewiesen, die hiermit durch Bezugnahme in die vorliegende Offenbarung aufgenommen wird.

Die Lage der Tracking-Einheit wird bevorzugt durch die Position der Tracking-Einheit in einem vorbestimmten Bezugssystem bestimmt. Vorzugsweise wird als Bezugssystem ein Bezugssystem verwendet, in dem die Kameraeinheit ruht. Insbesondere wird die Lage der Tracking-Einheit durch die Positionen der Marker, insbesondere der Mittelpunkte der Marker in einem Bezugssystem, bestimmt. Die Positionen können z.B. mit kartesischen Koordinaten oder Kugelkoordinaten beschrieben werden. Die relative Lage von einem Teil (z.B. Kameraeinheit oder Tracking-Einheit) zu einem anderen Teil (z.B. Tracking-Einheit) kann insbesondere durch Raumwinkel und/oder Abstände und/oder Koordinaten (in einem Bezugssystem) und/oder Vektoren beschrieben werden und wird vorzugsweise aus den die Lage beschreibenden Positionen z.B. mittels eines Programms errechnet, das auf einem Computer läuft.

Das erfindungsgemäße System weist weiterhin eine Recheneinheit auf, die mit der Kameraeinheit verbunden ist. Diese Verbindung kann über ein Kabel oder auch kabellos (engl. "wireless") erfolgen. Die erfindungsgemäße Recheneinheit ist angepasst, um aus mittels der Detektionseinheit gewonnenen Daten eine erste relative Lage der Landmarken relativ zu der ersten Tracking-Einheit zu bestimmen. Die gewonnenen Daten können direkt in die Recheneinheit eingespeist werden, indem z.B. eine dauerhafte Verbindung (über Kabel oder kabellos) zwischen Recheneinheit und Detektionseinheit besteht. Alternativ ist es möglich, die Daten mit der Detektionseinheit separat aufzuzeichnen und z.B. auf einem Datenträger zwischenzuspeichern, der später dann von der Recheneinheit ausgelesen wird. Erfindungsgemäß ist die Recheneinheit auch angepasst, um eine zweite relative Lage der Landmarken relativ zu der zweiten Tracking-Einheit basierend auf der ersten Lage zu berechnen, so dass zusätzliche navigierte Behandlungsgeräte während der Operation durch Bezugnahme auf die zweite Tracking-Einheit exakt positioniert werden können. Ein navigiertes Behandlungsgerät ist ein Behandlungsgerät, das seinerseits mit einer an sich bekannten Tracking-Einheit ausgestattet ist. Diese Tracking-Einheit ist an dem navigierten Behandlungsgerät an einer Stelle angebracht, deren Lage relativ zum Behandlungsgerät dem System bekannt ist (entweder durch Vordefinition oder durch erneute Kalibrierung vor jeder Anwendung). Aus der Kenntnis der Position der Tracking-Einheit kann dann auf die Position des Behandlungsgerätes rückgeschlossen werden.

Der hierin verwendete Begriff "relative Lage" bzw. der Ausdruck "Lage eines Teils A relativ zu einem Teil B" umfasst also den Begriff der relativen Positionen zwischen zwei Teilen, insbesondere zwischen den Tracking-Einheiten und/oder deren Markern oder zwischen einer Tracking-Einheit (oder deren Markern) und der Kameraeinheit. Insbesondere werden Schwerpunkte oder Mittelpunkte der Teile als punktförmiger Bezugspunkt zum Festlegen einer Position gewählt. Ist die Position eines Teils in einem Bezugssystem bekannt, so kann basierend auf der relativen Lage zweier Teile aus der Position eines der zwei Teile die Position des anderen der zwei Teile berechnet werden.

Die Tracking-Einheit gemäß der vorliegenden Erfindung umfasst vorzugsweise mindestens zwei Marker, insbesondere und bevorzugt drei Marker und kann natürlich auch mehr als drei Marker umfassen. Die Abmessungen der Marker und die relativen Lagen der Marker zueinander sind bekannt und liegen insbesondere als vorbekannte Daten einer Datenverarbeitungseinrichtung vor oder wurden durch einen Kalibriervorgang bestimmt. Vorzugsweise ist auch die Form der Marker bekannt.

Gemäß dem erfindungsgemäßen System ist es besonders vorteilhaft, dass zusätzliche navigierte Behandlungsgeräte während der Operation unter Bezugnahme auf die zweite Tracking-Einheit exakt positioniert werden können. Normalerweise wird beim Einsatz navigierter Behandlungsgeräte- lediglich Bezug auf eine erste Tracking-Einheit genommen. Durch Bezugnahme auf diese erfolgt die Navigation, d.h. Positionierung des Behandlungsgerätes. Wird die erste Tracking-Einheit dann entfernt, so endet der navigations-unterstützte Teil der Operation. Im Gegensatz dazu wird gemäß dem erfmdungsgemäßen System zwecks weitergehender Navigations-Unterstützung von der ersten Tracking-Einheit zur zweiten Tracking-Einheit gewechselt. Mit dem Entfernen der ersten Tracking-Einheit, z.B. weil ein Knochenabschnitt entfernt werden soll, an dem die erste Tracking-Einheit angebracht ist, ist erfindungsgemäß nun nicht länger ein Informationsverlust verbunden. Stattdessen bleiben alle Kenntnisse über die Topographie des Knochens (beschrieben durch die Landmarken) und optional auch über die Position eines zusätzlich navigierten Behandlungsgerätes bezogen auf diese Topographie erhalten. Der Wechsel des Bezugssystems von der ersten Tracking-Einheit hin zur zweiten Tracking-Einheit wird dadurch ermöglicht, dass die relative Lage der Tracking-Einheiten zueinander bekannt ist. Diese Kenntnis wird mittels der Kameraeinheit gewonnen, die beide Tracking-Einheiten gleichzeitig erkennt. Die Positionsdaten werden dann von der Recheneinheit ausgewertet. Konkret ist die Recheneinheit angepasst, um aus mittels der oben beschriebenen Detektionseinheit gewonnenen Daten eine erste relative Lage der Landmarken relativ zu der ersten Tracking-Einheit zu bestimmen, und um eine zweite relative Lage der Landmarken relativ zu der zweiten Tracking-Einheit basierend auf der ersten relativen Lage zu berechnen.

Vorteilhaft ist es so, dass zusätzliche navigierte Behandlungsgeräte während der Operation wählbar durch Bezugnahme auf die erste Tracking-Einheit oder durch Bezugnahme auf die zweite Tracking-Einheit exakt positioniert werden können. Es ist also so, dass eine Umschaltfunktion vorgesehen ist. In diesem Fall wird die Umschaltfunktion -betätigt oder ausgewählt, bevor die erste Tracking-Einheit aus dem System entfernt wird, z.B. weil während der Operation nun ein Knochenabschnitt entfernt werden soll, auf dem sich die erste Tracking-Einheit befindet.

Der Terminus "durch Bezugnahme auf eine Tracking-Einheit" bedeutet, dass in dem Rechenprozess, in dem die Position des navigierten Behandlungsgerätes bestimmt wird, auf die Position der Tracking-Einheit zurückgegriffen wird. Dieser Rückgriff kann in der Bezugnahme auf absolute Positionsdaten der Tracking-Einheit bestehen (z.B. Positionsdaten der Tracking-Einheit im Bezugssystem der Kameraeinheit), es ist aber auch möglich, dass auf die relative Lage der Landmarken relativ zu der Tracking-Einheit zurückgegriffen wird. Der Fachmann ist in der Lage, im Ergebnis äquivalente Rechenroutinen zu entwickeln und zu implementieren, die die "Bezugnahme auf die Tracking-Einheit" unterschiedlich umsetzen.

Vorteilhaft umfasst das erfindungsgemäße System ein zusätzliches navigiertes Behandlungsgerät. Dabei kann es sich um ein beliebiges zusätzliches navigiertes Behandlungsgerät handeln. Vorteilhaft ist das System mit einer navigierten Räumfräse ausgestattet (engl. "reamer"). Mit einer navigierten Räumfräse ist es möglich, den Knochen, an dem eine Prothese angebracht werden soll, exakt auszufräsen. Unter einem navigierten Behandlungsgerät wird ein Behandlungsgerät - wie oben ausgeführt - verstanden, das navigations-unterstützt während der Operation eingesetzt wird. Zu diesem Zweck ist das navigierte Behandlungsgerät mit einer Tracking-Einheit versehen, die mit zwei oder mehr aktiven oder passiven Markern ausgestattet ist. Diese werden mit der Kameraeinheit erkannt, und deren jeweilige Lage wird bestimmt. Gleichzeitig wird mit der Kameraeinheit -neben der Tracking-Einheit des Behandlungsgerätes eine weitere Tracking-Einheit, bevorzugt die zweite Tracking-Einheit, erkannt, um die jeweilige Lage der Tracking-Einheiten zu bestimmen. Aus der relativen Lage der zwei Tracking-Einheiten zueinander wird dann mittels der Recheneinheit die Position des navigierten Behandlungsgerätes bestimmt. Hinsichtlich der verwendbaren Tracking-Einheit und hinsichtlich der verwendeten Marker wird auf die obigen Ausführungen über die erste und zweite Tracking-Einheit und deren Marker verwiesen.

Vorteilhaft weist das zusätzliche Behandlungsgerät einen navigierten Adapter auf, d.h. das Behandlungsgerät weist einen Adapter auf, an dem die Tracking-Einheit des Behandlungsgerätes angebracht ist. Die geometrischen Abmessungen des Adapters sind so beschaffen, dass das zusätzliche Behandlungsgerät unabhängig von seiner konkreten Größe während der Operation exakt positionierbar ist. Die während einer Operation verwendeten Behandlungsgeräte können einerseits nach Typ und andererseits nach Größe unterschieden werden. Handelt es sich um navigierte Behandlungsgeräte, so muss- die geometrische Abmessung des Behandlungsgerätes zur Navigation genau bekannt sein. Genauer ist es notwendig, die Position der Tracking-Einheit am Behandlungsgerät genau zu kennen, so dass aus der Position der Tracking-Einheit rückgeschlossen werden kann auf die Position des aktiven Teils des Behandlungsgerätes, z.B. auf die exakte Position der Spitze einer Räumfräse. Ein erfindungsgemäßer Adapter -sorgt dafür, dass der Abstand und die Richtung zwischen der Tracking-Einheit und der aktiven Behandlungsposition des navigierten Behandlungsgerätes immer gleichbleibt. Die Tracking-Einheit des navigierten Behandlunsgerätes befindet sich an dem Adapter (navigierter Adapter). Der Adapter kann auf das Behandlungsgerät z.B. aufgeschoben oder aufgeschraubt werden. Wichtig ist dabei eine orientierungsfeste Verbindung zwischen Adapter und Behandlungsgerät.

Das erfindungsgemäße System für navigations-unterstützte Schulteroperationen weist ein Tracking-Einheit-Befestigungsmittel auf, an dem die zweite Tracking-Einheit befestigt ist und das am Knochen befestigbar ist. Bei dem Tracking-Einheit-Befestigungsmittel kann es sich z.B. um einen Stab handeln. Dieser Stab ist bevorzugt linear, d.h. ohne Krümmungen, ausgebildet und weist einen nicht runden Durchmesser, d. h. z. B. einem ovalen oder mehreckigen Durchmesser, auf. Das Tracking-Einheit-Befestigungsmittel kann am Knochen dadurch befestigbar sein, dass es in eine schmale Öffnung, die in den Knochen hineingebohrt worden ist, hineingesteckt- wird. Es ist möglich, dass das Tracking-Einheit-Befestigungsmittel einstückig oder mehrstückig ausgebildet ist.

Die zweite Tracking-Einheit ist abnehmbar an dem Tracking-Einheit-Befestigungsmittel derart befestigt, dass die Position der zweiten Tracking-Einheit, die diese vor dem Abnehmen inne hat, bei einem Wiederanbringen der Tracking-Einheit am Tracking-Einheit-Befestigungsmittel erneut eingenommen wird. Dies kann z.B. dadurch erreicht werden, dass zwischen dem Tracking-Einheit-Befestigungsmittel und der zweiten Tracking-Einheit eine Führung vorgesehen ist. Somit kann die zweite Tracking-Einheit an dieser Führung entlang auf das Tracking-Einheit-Befestigungsmittel z.B. aufgeschoben werden. Die Führung kann in Form einer einfachen Schiene vorgesehen sein. Alternativ ist es möglich, dass die zweite Tracking-Einheit in das Tracking-Einheit-Befestigungsmittel hineingesteckt wird oder aber über dieses geschoben wird. Zu diesem Zweck ist entweder die zweite Tracking-Einheit oder das Tracking-Einheit-Befestigungsmittel teilweise hohl ausgebildet. Das Vorsehen einer abnehmbaren zweiten Tracking-Einheit in der oben beschriebenen Form hat den Vorteil, dass es möglich ist, zeitweise die zweite Tracking-Einheit während der Operation zu entfernen und sie anschließend wieder anzubringen, ohne dass ein Informationsverlust während der Operation entsteht. Beispielsweise kann es aufgrund von Platzmangel notwendig sein, die zweite Tracking-Einheit kurzzeitig zu entfernen. Wichtig ist in diesem Fall natürlich die Reproduzierbarkeit der Position, die die zweite Tracking-Einheit ursprünglich eingenommen hat.

Das erfindungsgemäße System weist eine Schneidführung (engl. "cutting guide") auf, die in einer gewünschten Orientierung zu dem navigierten Tracking-Einheit-Befestigungsmittel ausrichtbar ist. Diese Schneidführung kann z.B. über das Tracking-Einheit-Befestigungsmittel geschoben werden. Damit dies problemlos erfolgen kann, kann es ebenfalls notwendig sein, die zweite Tracking-Einheit temporär zu entfernen.

Es ist möglich, dass das erfindungsgemäße System für navigations-unterstützte Schulteroperationen ein weiteres Tracking-Einheit-Befestigungsmittel aufweist, an dem die erste Tracking-Einheit befestigt ist und das am Knochen befestigbar ist. So kann die erste Tracking-Einheit an einem ersten Tracking-Einheit-Befestigungsmittel und die zweite Tracking-Einheit an einem zweiten Tracking-Einheit-Befestigungsmittel angebracht sein. Dabei handelt es sich bei dem ersten und zweiten Tracking-Einheit-Befestigungsmittel bevorzugt um separate Einheiten, die jeweils für sich am Knochen befestigbar sind.

Vorteilhaft weist die Schneidführung eine Grenzfläche auf, um an dem Tracking-Einheit-Befestigungsmittel entlang zu gleiten. Auf diese Weise ist es möglich, mit einem bevorzugt navigierten Behandlungsgerät einen exakten Schnitt am Knochen auszuführen.

Vorteilhaft ist die erste Tracking-Einheit des erfindungsgemäßen Systems angepasst, um extramedullär am Knochen befestigt zu werden. Diese -Befestigungsform ist verhältnismäßig einfach zu bewerkstelligen. Des Weiteren hat sie den Vorteil, den Knochen nur so weit zu verletzen, -dass sämtliche Stellen, die von Schrauben -oder Klemmen beschädigt worden sind, während der Operation entfernt werden können.

Vorteilhaft umfasst das erfindungsgemäße System eine navigierte Manschette, die um die humerale Komponente herum befestigbar ist. Diese navigierte Manschette wird im Allgemeinen außen auf der Haut des Oberarms angebracht. Die navigierte Manschette ist mit einer Tracking-Einheit ausgestattet. Mittels der Kameraeinheit ist es möglich, die Tracking-Einheit der Manschette und die zweite Tracking-Einheit gleichzeitig zu erkennen, um deren jeweilige relative Lage zu bestimmen. Wird nun die zweite Tracking-Einheit vom Knochen abmontiert, so ist es möglich, einen erneuten Bezugssystemwechsel von der zweiten Tracking-Einheit hin zu der navigierten Manschette durchzuführen, was es erlaubt, einen Analyse der Schultergelenksbeweglichkeit nach der Implantation durchzuführen.

Gemäß einem weiteren Aspekt, der nicht Gegenstand der beanspruchten Erfindung ist, wird ein Navigationsverfahren zur Positionierung navigierter Behandlungsgeräte bezüglich eines Knochens bereitgestellt. Mithilfe dieses Verfahrens ist es möglich, die navigierten Behandlungsgeräte bezüglich des Knochens derartig exakt zu positionieren, dass z.B. eine Bohrung, Fräsung oder ein Schnitt, der mittels des navigierten Behandlungsgerätes ausgeführt werden soll,- exakt ausgeführt werden kann, was sich positiv auf ein Behandlungsergebnis auswirken kann.

Unter einem navigierten Behandlungsgerät wird - wie bereits oben beschrieben - ein Behandlungsgerät verstanden, das mit einer Tracking-Einheit versehen ist, so dass die Positionsbestimmung des Behandlungsgerätes bzw. der behandlungsaktiven Position des Gerätes (z.B. Spitze eines Bohrers, wenn es sich um einen Bohrer handelt) genau durchgeführt werden kann.

Das erfindungsgemäße Navigationsverfahren weist die folgenden Schritte auf: Zunächst werden Landmarken des Knochens detektiert. Zum Detektieren können herkömmliche bekannte Detektionseinheiten wie z.B. Pointer verwendet werden. Es können einige wenige charakteristische Punkte eines Knochens detektiert werden, es ist aber auch möglich, einen vollständigen Scan der Oberfläche eines Knochens durchzuführen.

Anschließend wird in einem nächsten Schritt eine erste relative Lage der detektierten Landmarken relativ zu einem ersten Referenzsystem mit mindestens zwei Referenzpunkten ermittelt. Unter dem Begriff "relative Lage" sei dasselbe verstanden, wie oben in Zusammenhang mit dem erfindungsgemäßen System erläutert wurde. Bei dem ersten Referenzsystem mit mindestens zwei Referenzpunkten handelt es sich bevorzugt um eine Tracking-Einheit wie oben beschrieben.

In einem nächsten Verfahrensschritt werden mittels einer Kameraeinheit gleichzeitig mindestens zwei Referenzpunkte des ersten Referenzsystems und mindestens zwei Referenzpunkte eines zweiten Referenzsystems detektiert. Bei dem zweiten Referenzsystem mit mindestens zwei Referenzpunkten handelt es sich bevorzugt um eine Tracking-Einheit wie oben beschrieben.

Bevorzugt werden das erste und zweite Referenzsystem durch eine erste und zweite Tracking-Einheit gebildet, wie letztere im Zusammenhang mit dem beanspruchten System beschrieben worden sind.

In einem nächsten Verfahrensschritt wird eine zweite relative Lage der Landmarken relativ zu dem zweiten Referenzsystem basierend auf der ersten relativen Lage berechnet, so dass die zu navigierenden Behandlungsgeräte durch eine Bezugnahme auf das zweite Referenzsystem exakt positioniert werden können. Der Begriff "relative Lage" ist erneut wie oben definiert zu verstehen.

Entscheidend für das Navigationsverfahren ist es, dass eine Umrechnung der relativen Lage der Landmarken relativ zum ersten Referenzsystem hin zu einer relativen Lage der Landmarken relativ zu dem zweiten Referenzsystem erfolgt. Auf diese Weise wird ein Informationsverlust während des Navigationsverfahrens vermieden, wenn das erste Referenzsystem gleich aus welchen Gründen zur Positionsbestimmung des navigierten Behandlungsgerätes nicht mehr zur Verfügung steht.

Vorteilhaft weist das Navigationsverfahren den weiteren Verfahrensschritt des Positionierens des navigierten Behandlungsgerätes durch eine Bezugnahme auf das zweite Referenzsystem auf. Diese Positionierung erfolgt dann wieder in an sich bekannter Weise. Insbesondere wird die Tracking-Einheit des navigierten Behandlungsgerätes gleichzeitig mit dem zweiten Referenzsystem in Form der zweiten Tracking-Einheit von der Kameraeinheit erfasst, und die relative Lage zwischen navigiertem Behandlungsgerät und dem zweiten Referenzsystem wird berechnet.

Im Folgenden soll die vorliegende Erfindung unter Bezugnahme auf die beigefügten Figuren eingehender beschrieben werden. Es zeigen:
- Figur 1: eine Tracking-Einheit, die am Oberarmknochenkopf befestigt ist.
- Figur 2: die Detektion von Landmarken auf dem Oberannknochenkopf.
- Figur 3: einen navigierten Stab, der in einen ausgefrästen humeralen Kanal hineingesteckt ist, mit einer zweiten Tracking-Einheit, sowie eine gleichzeitig angebrachte erste Tracking-Einheit.
- Figur 4: eine Schneidführung, die relativ zu dem navigierten Stab orientiert ist, und eine Schnittfläche am Oberarmknochen.

Um eine navigations-unterstützte Schulteroperation durchführen zu können, muss zunächst eine so genannte Registrierung der humeralen Komponente, d.h. des Oberarmknochens durchgeführt werden. Zu diesem Zweck wird eine erste Tracking-Einheit 1 in herkömmlicher Weise am Oberarmknochenkopf- 2 (engl. "humeral head") befestigt.

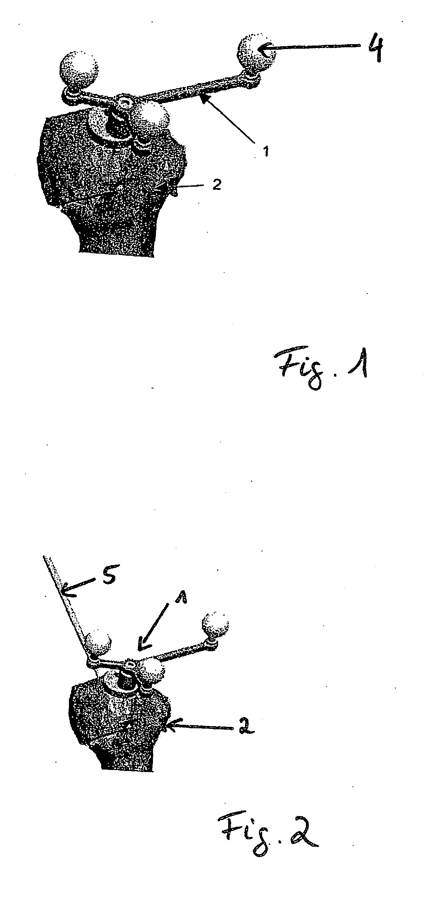

Die Befestigung einer ersten Tracking-Einheit 1 am Oberarmknochenkopf 2 ist in Figur 1 dargestellt. Die Befestigung der Tracking-Einheit 1 erfolgt an einem seitlichen Bereich des Oberarmknochenkopfes 2. Im vorliegenden Fall erfolgt die Befestigung- unter Verwendung von Schrauben. Die Tracking-Einheit 1 weist drei passive Marker 4 auf, deren relative Positionen zueinander genau bekannt sind. Durch die drei Marker 4 der ersten Tracking-Einheit 1 wird ein erstes Referenzsystem definiert.

Die erste Tracking-Einheit 1 ist an einem seitlichen Bereich des Oberarmknochenkopfes 2 angebracht. Dieser Bereich wird während der Operation entfernt. Das Einbringen von Schrauben in den Knochen -verursacht grundsätzlich Verletzungen, die zu Instabilitäten führen können. Da jedoch in diesem Ausführungsbeispiel dieser Bereich des Knochens, in den die Schrauben eingegriffen haben, während der Operation entfernt wird, wird auf diese Weise das Destabilisierungsrisiko minimiert.

Um die Oberflächenpositionen (im Folgenden "Landmarken") des Oberarmknochenkopfes 2 zu registrieren, wird ein Pointer 5 eingesetzt. Mit diesem wird die Oberfläche des Oberarmknochenkopfes abgetastet, und die Landmarken auf dem Knochen 2 werden somit detektiert. Dies ist in Figur 2 dargestellt. Unter Bezugnahme auf die Tracking-Einheit 1 wird Information über die Knochentopographie gesammelt. Es ist somit möglich, aus mittels der Detektionseinheit gewonnenen Daten eine erste relative Lage der Landmarken relativ zu der ersten Tracking-Einheit zu bestimmen.

Anschließend wird in der beschriebenen Ausführungsform ein humeraler Bohrer (engl. "humeral drill") eingesetzt, bei dem es sich bevorzugt um ein navigiertes Behandlungsgerät handelt. Der humerale Bohrer kann in navigierter Form relativ zu der ersten Tracking-Einheit 1 am Oberarmknochenkopf 2 orientiert werden, indem eine Tracking-Einheit an dem Bohrer befestigt wird.

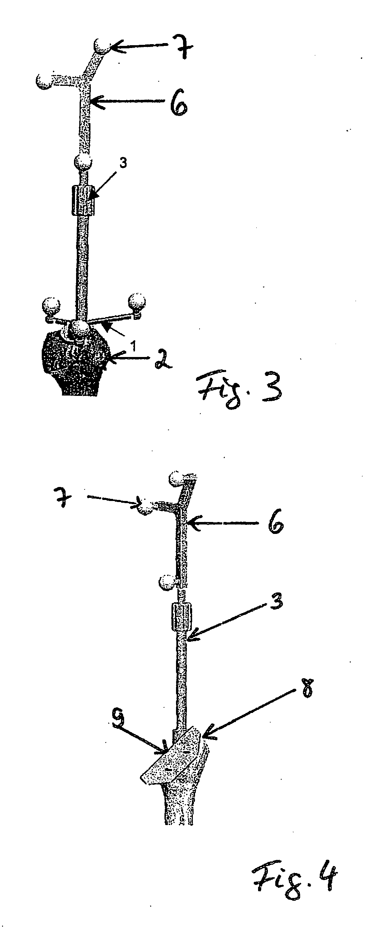

Nun wird der humerale Kanal navigiert ausgeräumt. Der Bohrer wird entfernt und durch einen Stab 3 ersetzt, der in den ausgeräumten humeralen Kanal hineingesteckt wird (siehe Figur 3). Dieser Stab 3 weist eine zweite Tracking-Einheit 6 auf, die im vorliegenden Fall über drei Marker 7 verfügt. Der Stab ist in dem Knochen rotationsgesichert befestigt. Wie aus Figur 3 hervorgeht, sind nun zu einem bestimmten Zeitpunkt zwei verschiedene Tracking-Einheiten an ein und demselben Knochen befestigt. Die Kameraeinheit erkennt die beiden Tracking-Einheiten gleichzeitig, und da die Landmarken relativ zu der ersten Tracking-Einheit bestimmt worden sind, kann das System diese Information umrechnen und in Beziehung zu der zweiten Tracking-Einheit setzen, die sich auf dem navigierten Stab 3 befindet.

Im Folgenden wird die erste Tracking-Einheit 1 entfernt, ohne dass dadurch gewonnene Information verloren würde. Dies ist in Figur 4 illustriert. Als Nächstes kann eine Schneidrührung 9 (engl. "cutting guide") in der gewünschten Orientierung relativ zu dem navigierten Stab 3 ausgerichtet werden und ein Teil des Oberarmknochenkopfes wird abgeschnitten.

Es ist möglich, spezielle Schneidführungen oder gewöhnliche Schneidführungen zu verwenden. Um die Verwendung von gewöhnlichen Schneidführungen zu ermöglichen, die normalerweise an einem Frässchaft (engl. "reamer shaft") befestigt werden, ist die zweite Tracking-Einheit 6 an dem Stab 3 abnehmbar ausgebildet, so dass die Schneidführung über den navigierten Stab 3 geschoben werden kann. Anschließend wird die zweite Tracking-Einheit 6 wieder befestigt, wobei sie ihre ursprüngliche Position, die sie auf dem Stab 3 innehatte, wieder einnimmt. Zu diesem Zweck ist die zweite Tracking-Einheit 6 verdrehsicher auf dem Stab 3 anbringbar.

Die folgende Vorbereitung der humeralen Komponente für den Einsatz des Implantates hängt von dem verwendeten Implantat und dem angewendeten Operationsverfahren ab. Im Allgemeinen wird nun ein Ausfräsen (engl. "broaching") durchgeführt. Vorteilhaft wird dazu ein zusätzliches navigiertes Behandlungsgerät eingesetzt, um eine definierte Orientierung der Ausfräsung (engl. "broaching") vorzunehmen. Die Positionierung dieses zusätzlichen navigierten Behandlungsgerätes wird unter Bezugnahme auf die zweite Tracking-Einheit durchgeführt.

Um den Einsatz von Behandlungsgeräten gleichen Typs, aber unterschiedlicher Größe mit niedrigem Aufwand zu ermöglichen, wird ein Adapter eingesetzt, dessen geometrische Abmessungen so beschaffen sind, dass das zusätzliche Behandlungsgerät unabhängig von seiner konkreten Größe während der Operation exakt positionierbar ist.

Anhand der Figuren wurde eine bevorzugte Ausführungsform der Erfindung beschrieben, die bei navigations-unterstützten Schulteroperationen zum Einsatz kommen kann. Es ist für den Fachmann jedoch ohne Weiteres ersichtlich, dass das erfindungsgemäße System und das erfindungsgemäße Navigationsverfahren auch bei anderen Operationsarten eingesetzt werden können. Das beschriebene Ausführungsbeispiel ist insofern nicht schutzeinschränkend auszulegen.

## Patentansprüche

1. System für navigations-unterstützte Schulteroperationen, in denen die humerale Komponente (2) teilweise durch eine Prothese ersetzt wird, das folgendes aufweist:
• eine erste Tracking-Einheit (1), die angepasst ist, um an einem Abschnitt eines Knochens (2) befestigt zu werden, der während der Operation entfernt werden soll;
• eine Detektionseinheit (5), die angepasst ist, um Landmarken auf dem Knochen (2) zu detektieren;
• eine zweite Tracking-Einheit (6), die angepasst ist, um an einem anderen Abschnitt desselben Knochens (2) angebracht zu werden;
• eine Kameraeinheit, die die erste (1) und die zweite (6) Tracking-Einheit gleichzeitig erkennt, um deren jeweilige Lage zu bestimmen;
• eine Recheneinheit, die mit der Kameraeinheit verbunden ist, und die angepasst ist, um
○ aus mittels der Detektionseinheit (5) gewonnenen Daten eine erste relative Lage der Landmarken relativ zu der ersten Tracking-Einheit (1) zu bestimmen, und
○ eine zweite relative Lage der Landmarken relativ zu der zweiten Tracking-Einheit (6) basierend auf der ersten relativen Lage zu berechnen,
so dass zusätzliche navigierte Behandlungsgeräte während der Operation durch Bezugnahme auf die zweite Tracking-Einheit (6) exakt positioniert werden können,
• ein Tracking-Einheit-Befestigungsmittel (3), an dem die zweite Tracking-Einheit (6) befestigt ist und das am Knochen (2) befestigbar ist; und
• eine Schneidführung, die in einer gewünschten Orientierung zu dem navigierten Tracking-Einheit-Befestigungsmittel (3) ausrichtbar ist,
**dadurch gekennzeichnet, dass**
die zweite Tracking-Einheit (6) an dem Tracking-Einheit-Befestigungsmittel (3) abnehmbar ausgebildet ist, so dass die Schneidführung über das Tracking-Einheit-Befestigungsmittel (3) geschoben werden kann.

2. System gemäß Anspruch 1, wobei durch Verwendung einer Umschaltfunktion zusätzliche navigierte Behandlungsgeräte während der Operation wählbar durch Bezugnahme auf die erste Tracking-Einheit (1) oder durch Bezugnahme auf die zweite Tracking-Einheit (6) exakt positioniert werden können.

3. System gemäß einem der Ansprüche 1 oder 2 mit einem zusätzlichen navigierten Behandlungsgerät, insbesondere mit einer navigierten Räumfräse.

4. System gemäß Anspruch 3, wobei das zusätzliche Behandlungsgerät einen navigierten Adapter aufweist, dessen geometrische Abmessungen so beschaffen sind, dass das zusätzliche Behandlungsgerät unabhängig von seiner konkreten Größe während der Operation exakt positionierbar ist.

5. System gemäß einem der Ansprüche 1 bis 4, wobei die zweite Tracking-Einheit (6) abnehmbar an dem Tracking-Einheit-Befestigungsmittel (3) derart befestigt ist, dass die Position der zweiten Tracking-Einheit (6), die diese vor dem Abnehmen innehat, bei einem Wiederanbringen der Tracking-Einheit (6) am Tracking-Einheit-Befestigungsmittel (3) erneut eingenommen wird.

6. System gemäß Anspruch 5, wobei zwischen dem Tracking-Einheit-Befestigungsmittel (3) und der zweiten Tracking-Einheit (6) eine Führung vorgesehen ist.

7. System gemäß einem der Ansprüche 1, 5 oder 6, wobei die Schneidführung eine Grenzfläche aufweist, um an dem Tracking-Einheit-Befestigungsmittel (3) entlang zu gleiten.

8. System gemäß einem der Ansprüche 1 bis 7, wobei die erste Tracking-Einheit (1) angepasst ist, um extramedulär am Knochen (2) befestigt zu werden.

9. System gemäß einem der Ansprüche 1 bis 8 mit einer navigierten Manschette, die um die humerale Komponente (2) herum befestigbar ist.

## Claims

1. A system for navigation-assisted shoulder operations in which the humeral component (2) is partially replaced with a prosthesis, comprising:
• a first tracking unit (1) which is configured to be fastened to a section of a bone (2) which is to be removed during the operation;
• a detection unit (5) which is configured to detect landmarks on the bone (2);
• a second tracking unit (6) which is configured to be attached to another section of the same bone (2);
• a camera unit which simultaneously detects the first (1) and second (6) tracking unit, in order to determine their respective location;
• a computational unit which is connected to the camera unit and is configured to:
○ determine a first relative location of the landmarks relative to the first tracking unit (1) from data acquired by means of the detection unit (5); and
○ calculate a second relative location of the landmarks relative to the second tracking unit (6) on the basis of the first relative location,
such that additional navigated treatment apparatus can be exactly positioned during the operation by referring to the second tracking unit (6);
• a tracking unit fastening means (3), to which the second tracking unit (6) is fastened and which can be fastened to the bone (2); and
• a cutting guide which can be aligned in a desired orientation with respect to the navigated tracking unit fastening means (3),
**characterised in that**
the second tracking unit (6) is detachably formed on the tracking unit fastening means (3) such that the cutting guide can be slid over the tracking unit fastening means (3).

2. The system in accordance with claim 1, wherein by using a switching function, additional navigated treatment apparatus can be exactly positioned during the operation by referring optionally to the first tracking unit (1) or the second tracking unit (6).

3. The system in accordance with any one of claims 1 or 2, comprising an additional navigated treatment apparatus, in particular a navigated reamer.

4. The system in accordance with claim 3, wherein the additional treatment apparatus comprises a navigated adaptor whose geometric dimensions are designed such that the additional treatment apparatus can be exactly positioned during the operation, irrespective of its specific size.

5. The system in accordance with any one of claims 1 to 4, wherein the second tracking unit (6) is detachably fastened to the tracking unit fastening means (3) such that the position of the second tracking unit (6) which the second tracking unit occupies before being detached is again occupied when the tracking unit (6) is reattached to the tracking unit fastening means (3).

6. The system in accordance with claim 5, wherein a guide is provided between the tracking unit fastening means (3) and the second tracking unit (6).

7. The system in accordance with any one of claims 1, 5 or 6, wherein the cutting guide comprises a boundary area for sliding along the tracking unit fastening means (3).

8. The system in accordance with any one of claims 1 to 7, wherein the first tracking unit (1) is configured to be fastened to the bone (2) outside of the medulla.

9. The system in accordance with any one of claims 1 to 8, comprising a navigated cuff which can be fastened around the humeral component (2).

## Revendications

1. Système pour des opérations de l'épaule assistées par navigation, au cours desquelles le composant huméral (2) est partiellement remplacé par une prothèse, comportant les éléments suivants :
• une première unité de repérage (1) adaptée pour être fixée sur une partie d'un os (2) qui doit être retirée lors de l'opération,
• une unité de détection (5) adaptée pour détecter des points de repère sur l'os (2),
• une seconde unité de repérage (6) adaptée pour être appliquée sur une autre partie du même os (2),
• une unité de caméra qui détecte simultanément les première (1) et seconde (6) unités de repérage afin de déterminer leur position respective,
• une unité de calcul reliée à l'unité de caméra et adaptée pour :
° déterminer une première position relative des points de repère par rapport à la première unité de repérage (1) à partir de données acquises au moyen de l'unité de détection (5), et
° calculer une seconde position relative des points de repère par rapport à la seconde unité de repérage (6) sur la base de la première position relative,
de telle sorte que des appareils supplémentaires de traitement avec navigation peuvent être positionnés de manière précise pendant l'opération en se référant à la seconde unité de repérage (6),
• des moyens de fixation d'unité de repérage (3) sur lesquels est fixée la seconde unité de repérage (6) et qui peuvent être fixés sur l'os (2), et
• un guide de coupe qui peut être aligné dans une orientation voulue par rapport aux moyens de fixation d'unité de repérage avec navigation (3),
**caractérisé en ce que**
la seconde unité de repérage (6) est configurée de manière amovible sur les moyens de fixation d'unité de repérage (3) de telle sorte que le guide de coupe peut être déplacé par l'intermédiaire des moyens de fixation d'unité de repérage (3).

2. Système selon la revendication 1, dans lequel l'utilisation d'une fonction de commutation permet de positionner précisément des appareils supplémentaires de traitement avec navigation de manière sélective pendant l'opération en référence à l'unité de repérage (1) ou en référence à la seconde unité de repérage (6).

3. Système selon l'une quelconque des revendications 1 ou 2, comportant un appareil supplémentaire de traitement avec navigation, en particulier un alésoir avec navigation.

4. Système selon la revendication 3, dans lequel l'appareil de traitement supplémentaire comporte un adaptateur avec navigation dont les dimensions géométriques sont établies de telle sorte que l'appareil de traitement supplémentaire peut être positionné précisément lors de l'opération indépendamment de sa taille réelle.

5. Système selon l'une quelconque des revendications 1 à 4, dans lequel la seconde unité de repérage (6) est fixée de manière amovible sur les moyens de fixation d'unité de repérage (3) de telle sorte que la position de la seconde unité de repérage (6), que la seconde unité de repérage occupe avant d'être séparée, est de nouveau occupée lorsque l'unité de repérage (6) est refixée sur les moyens de fixation d'unité de repérage (3).

6. Système selon la revendication 5, dans lequel un guide est prévu entre les moyens de fixation d'unité de repérage (3) et la seconde unité de repérage (6).

7. Système selon l'une quelconque des revendications 1, 5 ou 6, dans lequel le guide de coupe présente une surface de délimitation afin de glisser le long des moyens de fixation d'unité de repérage (3).

8. Système selon l'une quelconque des revendications 1 à 7, dans lequel la première unité de repérage (1) est adaptée pour être fixée sur l'os (2) de manière extra médullaire.

9. Système selon l'une quelconque des revendications 1 à 8 comportant une manchette avec navigation qui peut être fixée autour du composant huméral (2).
